**Europäisches Patentamt**

**European Patent Office**

**Office européen des brevets**

(19)

(11) Veröffentlichungsnummer: **0 281 984 B1**

(12) # EUROPÄISCHE PATENTSCHRIFT

(45) Veröffentlichungstag der Patentschrift:
24.07.91 Patentblatt 91/30

(51) Int. Cl.⁵: **A61F 2/32, A61F 2/34**

(21) Anmeldenummer: **88103472.2**

(22) Anmeldetag: **05.03.88**

---

(54) **Hüftgelenk-Endoprothese.**

---

Teilanmeldung 90115738.8 eingereicht am 05/03/88.

(30) Priorität: **07.03.87 DE 3707428**

(43) Veröffentlichungstag der Anmeldung:
**14.09.88 Patentblatt 88/37**

(45) Bekanntmachung des Hinweises auf die Patenterteilung:
**24.07.91 Patentblatt 91/30**

(84) Benannte Vertragsstaaten:
**AT BE CH DE ES FR GB GR IT LI LU NL SE**

(56) Entgegenhaltungen:
**DE-A- 3 417 923**
**DE-A- 3 528 151**

(73) Patentinhaber: **Künne, Hermann, Dr.**
**Planweg 14**
**W-4782 Erwitte (DE)**

(72) Erfinder: **Künne, Hermann, Dr.**
**Planweg 14**
**W-4782 Erwitte (DE)**

(74) Vertreter: **Patentanwälte Dipl.-Ing. Bodo Thielking Dipl.-Ing. Otto Elbertzhagen**
**Gadderbaumer Strasse 20**
**W-4800 Bielefeld 1 (DE)**

---

**Beschreibung**

Die Erfindung betrifft eine Hüftgelenk-Endoprothese nach dem Oberbegriff des Anspruchs 1.

Bei einer bekannten Hüftgelenk-Endoprothese dieser Art (DE-A-3417923) ist die geschlitzte Hülse an ihrer Innenseite glattwandig und konisch ausgebildet. Die mit vorstehenden Rippen versehene Außenfläche verläuft parallel zur Innenfläche und ist im nicht eingesetzten Zustand ebenfalls konisch ausgebildet. Die Fixierung der geschlitzten Hülse in einer zylindrischen Aufnahmeöffnung des Beckens erfolgt dadurch, daß die Pfannenschale in einem außen kreiszylindrisch ausgebildeten Körper vorgesehen ist, der beim Eintreiben in die geschlitzte Hülse deren Spreizung und Festlegung in der Aufnahmeöffnung des Beckens vornimmt. Das Fixieren der geschlitzten Hülse durch ein Eintreiben des die Pfannenschale tragenden Stücks erweist sich als schwierig und belastend für den Patienten. Darüber hinaus besteht die Gefahr, daß die Pfannenschale sich beim Eintreiben in die geschlitzte Hülse verformt, wenn die Pfannenschale in einem Kunststoffkörper vorgesehen ist.

Bei einer anderen bekannten Hüftgelenk-Endoprothese (EP-B-0083708) ist die geschlitzte Hülse ebenfalls an ihrer Außenseite und Innenseite kegelstumpfförmig ausgebildet. In den kegeligen Innenhohlraum dieser Hülse ist ein konisch geformter Einsatz einpreßbar, der die Pfannenschale enthält.

Die konische Form des Pfannenteils der bekannten Hüftgelenk-Endoprothese ist nachteilig. Es ist schwierig, für ihren Einsatz eine passende konische Bohrung im Beckenknochen vorzusehen. Darüber hinaus ist es besonders schwierig, nachträgliche Lagekorrekturen des Pfannenteils in der Aufnahmeöffnung des Beckens vorzunehmen.

Ausgehend von dem als bekannt vorausgesetzten Stand der Technik liegt der Erfindung die Aufgabe zugrunde, eine Hüftgelenk-Endoprothese zu schaffen, die ein genaues und schonendes Einführen in den zugehörigen Aufnahmebereich des Knochens ermöglicht, wobei eine einwandfreie Justierung in der endgültigen Einbaulage sowie eine sichere und endgültige Fixierung in dieser Einbaulage erreicht werden soll.

Die Lösung dieser Aufgabe erfolgt mit den Merkmalen des Kennzeichnungsteils von Anspruch 1.

Weitere Merkmale ergeben sich aus den Unteransprüchen und der nachfolgenden Zeichnungsbeschreibung in Verbindung mit der Zeichnung.

Die erfindungsgemäße Hüftgelenk-Endoprothese erlaubt einen besonders schonenden Einbau und eine sichere endgültige Fixierung. Es ist möglich, daß der Patient bereits am Tag nach der Operation wieder aufstehen kann. Die Gefahr von Fettembolien wird reduziert. Ferner wird sicher ausgeschlossen, daß Knochen oder Knochenbereiche beim Einsetzen der Hüftgelenk-Endoprothese gesprengt werden könnten.

In der Zeichnung zeigen :

Figur 1 — eine Seitenansicht des Schaftteils der Prothese,
Figur 2 — eine Seitenansicht auf das Schaftteil gemäß Figur 1 in Richtung des Pfeils II,
Figur 3 — einen Querschnitt durch den unteren Bereich des Schaftteils ohne eingeführtes Spreizelement,
Figur 4 — den Schnitt durch das Schaftteil mit eingeführtem Spreizelement,
Figur 5 — eine perspektivische Darstellung einer ersten Ausführungsform des Pfannenteils in perspektivischer Explosionsdarstellung,
Figur 6 — eine zweite Ausführungsform des Pfannenteils in perspektivischer Explosionsdarstellung.

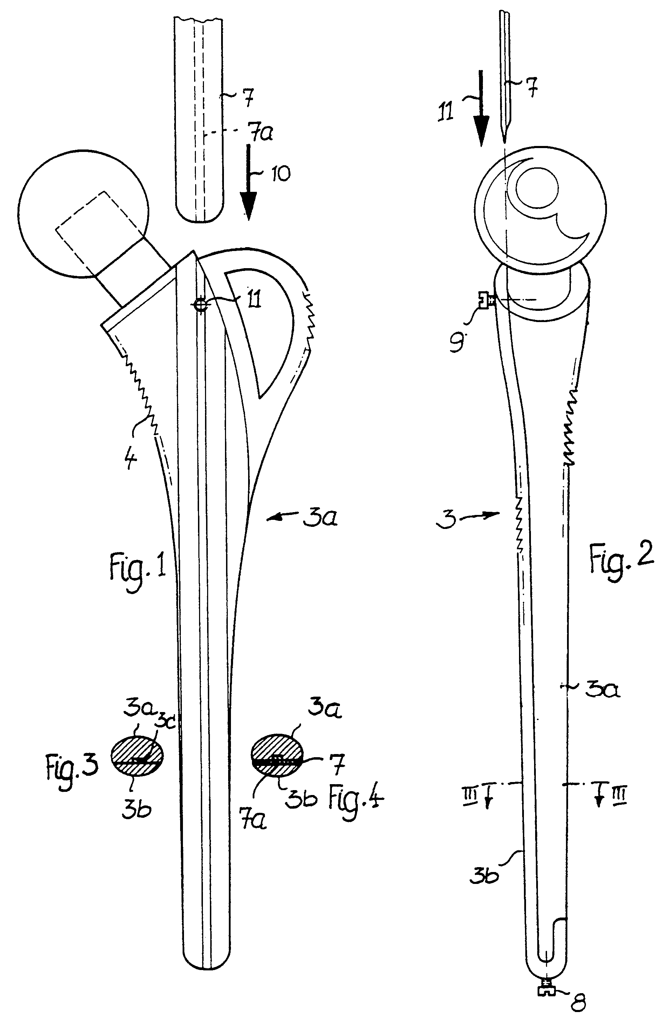

In den Figuren 1 und 2 ist der Schaftteil 3 der Hüftgelenk-Endoprothese sichtbar, wobei Figur 1 nur den Bereich 3a zeigt und im oberen Bereich ein Spreizelement 7, während Figur 2 die beiden Bereiche 3a und 3b im zusammengesetzten Zustand ohne eingeführtes Spreizelement zeigt.

Die beiden Bereiche 3a und 3b werden in der gemäß Figur 2 dargestellten Lage zusammengefügt in die Knochenhöhle des Oberschenkelknochens eingeführt. Dabei sind die beiden Bereiche 3a und 3b über die untere Schraube 8 miteinander verschraubt. Die obere Schraube 9 ist zunächst gelöst. Anschließend wird im Einsetzzustand des Schaftteils 3 das blattförmige Spreizelement 7, welches eine Rippe 7a aufweist, in Richtung des Pfeils 10 zwischen die beiden Bereiche 3a und 3b eingetrieben. Dabei gleitet die Rippe 7a in der Aufnahmenut 3c des Bereichs 3a. Wenn das Spreizelement in seiner Endposition liegt, ist dessen oberes Ende unterhalb der Schraube 9 angelangt. Danach kann die im Bereich 3b sitzende Schraube 9 in die Gewindebohrung 11 des Bereichs 3a eingeschraubt werden.

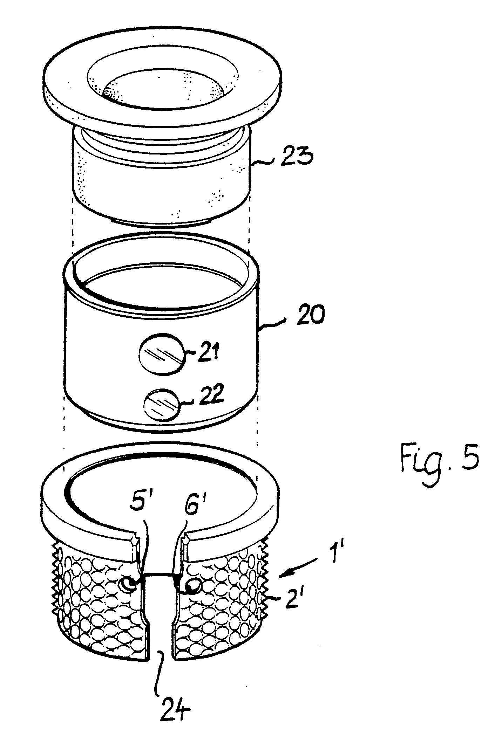

Die Vorsprünge 4 können auch andere als die dargestellte sägezahnartige Kontur haben. Sie können sich bei Bedarf über alle gewünschten Bereiche der Außenkontur des Schaftteils 3 erstrecken. Die Ausbildung und Größe der Vorsprünge kann unterschiedlich gewählt werden. Die beiden Schnittzeichnungen bei Figuren 3 und 4 sind der Einfachheit halber ohne Vorsprünge gezeichnet.

In Figur 5 ist der Pfannenteil dargestellt. Er besteht aus einem Ringkörper 1', einem metallischen Ring 20 und einer Kunststoffpfanne 23. Der Ringkörper 1' weist auf seinem Umfang raspelartig oder messerartig ausgebildete Vorsprünge 2' auf. In der Zeichnung ist nicht dargestellt, daß diese Vorsprünge, die auch eine andere Form und Orientierung aufweisen können, leicht abwärts geneigt sein können. Bei einer leichten Abwärtsneigung der messerartig ausgebildeten Vorsprünge hat das Spreizen des metallischen Ringbereichs 1' zur Folge, daß der Pfannenteil sich fest gegen den Boden der zylindrischen Aufnahmeöffnung legt und in dieser Lage gehalten wird.

Zum Einbau des Ringkörpers 1' wird die Trennfuge 24 mit einem Spezialwerkzeug, welches in die Montageöffnungen 5 und 6 eingesetzt wird, zunächst verringert und anschließend wieder vergrößert. In der gespreizten, vergrößerten Lage des Ringkörpers 1' ist die Trennfuge 24 am größten. In dieser Position haben sich die Vorsprünge 2' in die umgebende Knochenmasse eingedrückt. Ein geschlossener zylindrischer Ring 20 wird anschließend in den eingebauten Ringkörper 1' eingeschoben. Dabei liegen sich die Vorsprünge 22 und 21 an die einander gegenüberliegenden Stirnflächen der Trennfuge 24 an. Die Vorsprünge 22 und 21 und die Trennfuge 24 können so bemessen sein, daß beim Einschieben des Metallrings 20 eine endgültige und weitere Spreizung des Körpers 1' erfolgt. Im Metallring 20 sitzt die Kunststoffpfanne 23 mit einer Pfannenschale zur Aufnahme der Kugel des Schaftteils der Prothese.

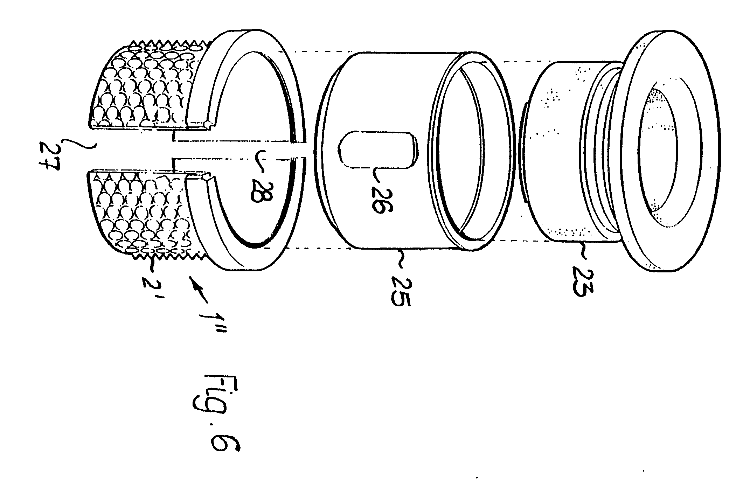

Bei der weiteren Ausführungsform gemäß Figur 6 ist ein Ringkörper 1' vorgesehen, der aus zwei sich zu einem Ring ergänzenden Hälften besteht. Die beiden Halbschalen sind entlang zweier einander gegenüberliegender Trennfugen 27 und 28 geteilt. Der Einsatz der beiden Halbschalen erfolgt mit in den Trennfugen aneinanderstoßenden Stirnflächen. Anschließend werden die beiden Halbschalen mittels eines Spezialwerkzeugs so weit wie möglich radial nach außen gepreßt. In der gespreizten Lage wird der geschlossene Metallring 25 mit der Kunststoffpfanne 23 eingeschoben oder leicht eingeschlagen. Dabei legt sich der als Distanzelement dienende Vorsprung 26 an die Stirnseiten der einander gegenüberliegenden Flanken der Trennfuge 27 an. Ein entsprechender Vorsprung, der nicht dargestellt ist, kann zum Auseinanderdrücken der Flanken der gegenüberliegenden Trennfuge 28 auf der gegenüberliegenden Seite des Metallrings 25 vorgesehen sein.

## Patentansprüche

1. Hüftgelenk-Endoprothese mit einem in einer Aufnahmeöffnung des Beckens fixierbaren Pfannenteil (1', 20, 23 ; 1", 25, 23) und einem im Oberschenkelknochen fixierbaren Schaftteil (3) mit Kugel, welche in einer Pfannenschale (23) abläuft, die in einer geschlitzten Hülse (1' ; 1") angeordnet ist, welche auf ihrer Oberfläche mit Abständen voneinander angeordnete, widerhakenartige Rastelemente (2') aufweist und als spreizbarer, metallischer, kreiszylindrischer Ringkörper (1' ; 1") ausgebildet ist, dadurch gekennzeichnet, daß ein in den Ringkörper (1' ; 1") einsetzbarer Metallring (20 ; 25) mit mindestens einem an seiner Außenseite angeordneten metallischen Distanzelement (21, 22 ; 26) vorgesehen ist, wobei im eingesetzten Zustand des Metallrings (20 ; 25) das Distanzelement (26) bzw. die Distanzelemente (21, 22) an den einander gegenüberliegenden Stirnflächen der Trennfuge (24 ; 27) des geschlitzten Ringkörpers (1' ; 1") anliegt bzw. anliegen und den Ringkörper (1', 1") in seiner gespreizten Einbaulage hält bzw. halten.

2. Hüftgelenk-Endoprothese nach Anspruch 1, dadurch gekennzeichnet, daß in den Metallring (20 ; 25) eine die Pfannenschale enthaltende Kunststoffpfanne (23) eingesetzt ist.

3. Hüftgelenk-Endoprothese nach Anspruch 1 oder 2, dadurch gekennzeichnet, daß der Ringkörper (1' ; 1") aus einer Titanlegierung besteht.

4. Hüftgelenk-Endoprothese nach einem oder mehreren der Ansprüche 1 bis 3, dadurch gekennzeichnet, daß benachbart zur Trennfuge (24) an beiden Teilen des Ringkörpers (1') Montageöffnungen (5' ; 6') vorgesehen sind.

## Claims

1. Hip joint endoprosthesis with a socket part (1', 20, 23 ; 1", 25, 23) fixable in a socket opening in the pelvis, and a shaft part (3) with ball fixable in the femur whereby the ball moves in a socket cup (23) which is mounted in a slit casing (1', 1") which has on its surface spaced barb-like detent elements (2') and which is designed as an expanding metal circular cylindrical annular body (1' ; 1") characterised in that a metal ring (20 ; 25) insertable in the annular body (1' ; 1") is provided with at least one metal spacer element (21, 22 ; 26) set on the outside wherein when the metal ring (20 ; 25) is inserted, the spacer element (26) or spacer elements (21, 22) adjoin(s) the opposing end faces of the dividing gap (24 ; 27) of the slit annular body (1' ; 1") and hold(s) the annular body (1', 1") in its expanded installation state.

2. Hip joint endoprosthesis according to claim 1 characterised in that a plastics socket (23) containing the socket cup is inserted in the metal ring (20 ; 25).

3. Hip joint endoprosthesis according to claim 1 or 2 characterised in that the ring body (1' ; 1") is made from a titanium alloy.

4. Hip joint endoprosthesis according to one or more of claims 1 to 3 characterised in that assembly openings (5' ; 6') are provided adjoining the dividing gap (24) on both parts of the annular body (1').


## Revendications

1. Endoprothèse d'articulation de hanche avec une pièce comprenant une cavité orbiculaire fixable dans un orifice de réception du bassin (1', 20, 23, 1", 25, 23) et un corps de prothèse (3), fixable dans le fémur pourvu d'une tête, se déplaçant dans une cavité orbiculaire (23), logée dans un manchon fendu (1' ; 1"), dont la surface présente des éléments de crantage (2'), genre barbes, disposés à intervalle les uns des autres, ce manchon fendu étant conçu sous forme de pièce annullaire, cylindrique circulaire (1' ; 1") caractérisée par le fait qu'une douille métallique (20 ; 25), insérable dans la pièce annullaire (1' ; 1") est prévue, laquelle présente, au moins un élément métallique (21, 22 ; 26) d'écartement sur sa face externe, l'élément d'écartement (26) ou les éléments d'écartement (21, 22) se pressant contre les parties frontales, se faisant face, du joint (24 ; 27) de la pièce annullaire fendue (1', 1") et maintenant la pièce annullaire (1', 1") dans sa position d'intégration écartée, lorsque la douille métallique (20 ; 25) est mise en place.

2. Endoprothèse d'articulation de hanche selon revendication 1, caractérisée par le fait qu'une pièce en matière plastique (23) contenant la cavité orbiculaire est insérée dans la douille métallique (20 ; 25).

3. Endoprothèse d'articulation de hanche selon revendication 1 ou 2, caractérisée par le fait que la pièce annulaire (1' ; 1") est exécutée en alliage de titan.

4. Endoprothèse d'articulation de hanche selon une ou plusieurs revendications 1 à 3, caractérisée par le fait que des ouvertures de montage (5' ; 6') sont prévues, à proximité du joint (24), dans les deux parties de la pièce annullaire (1').

Fig. 1

Fig. 2

Fig. 3

Fig. 4

Fig. 5

~23

~25

26

~28

Fig.6

1"

2'

27